# EUROPEAN PATENT SPECIFICATION

(11) **EP 4 101 417 B1**
(45) Date of publication and mention of the grant of the patent: **21.02.2024**
(21) Application number: 21382508.6
(22) Date of filing: 07.06.2021
(51) Int. Cl.: A61C 7/00, A61C 13/00, B33Y 50/02, G16H 50/50, A61C 9/00, A61C 1/08

(54) **DESIGN METHOD OF AN INTRAORAL DEVICE**
ENTWURFSVERFAHREN EINER INTRAORALEN VORRICHTUNG
PROCÉDÉ DE CONCEPTION D'UN DISPOSITIF INTRA-ORAL

(43) Date of publication of application: 14.12.2022
(73) Proprietor: Biotechnology Institute, I MAS D, S.L., 01005 Vitoria (ES)
(72) Inventor: ANITUA ALDECOA, Eduardo, 01005 VItoria (ES)
(74) Representative: Cueto, Sénida

(56) References cited:
- WO-A1-2018/038748
- US-A1- 2016 004 811
- US-A1- 2018 303 581

## Description

### Field of the invention

The invention refers to a method of designing an intraoral device through the use of a computer and a program or software for Computer-Aided Design, hereinafter CAD. Specifically, the method is for the design of intraoral devices, or oral splints, which are intended to be placed in the mouth of a user for the treatment of various jaw disorders such as bruxism, oral breathing or snoring and/or sleep apnoea, among others.

### Prior art

Currently, various intraoral or mandibular devices for mandibular release and/or advancement are known to treat bruxism and sleep apnoea during the patient's sleep.

These mandibular devices are made to meet the specific needs of the patient and are known as discharge or mandibular advancement splints. On the one hand, mandibular discharge splints are devices made of a non-metallic, rigid material that is placed in the patient's mouth, generally in the upper arch, and they prevent the upper dental pieces from being pressed against the lower dental pieces. Moreover, a splint keeps the maxillary bones (upper and lower) in the correct position so that they do not exert more force than necessary during the time the splint is in place, making the muscles relax and preventing them from tensing.

On the other hand, these mandibular devices or mandibular advancement splints can also cause slight advancement of the lower jaw, which prevents airway closure while the patient sleeps. This device is also made to measure for the patient, and is placed inside the patient's mouth in such a way that it comprises an upper area intended to be placed in the upper dental arch and a lower area intended to be placed in the lower dental arch. The mandibular advancement mechanisms are connected between the upper zone and the lower zone, positioned and tensed in such a way as to keep the lower zone advanced with respect to the upper zone. This causes a slight advancement of the jaw with respect to the upper jaw, compared to a resting position in which both portions would be one on top of the other for the patient's normal bite. This opens a larger space at the back of the oral cavity and thus facilitates the passage of air to and from the pharynx.

The mandibular devices defined above are designed and manufactured using CAD/CAM technology; CAD being Computer Aided Design and CAM being Computer Aided Manufacturing. This technology is widely known in the dental sector, where normally any dental element such as a crown, implant or splint is designed using CAD and printed using CAM or 3D printing.

The design of mandibular devices or dental elements using this computer-implemented CAD/CAM technology begins with an intraoral scan of the patient. An intraoral scanner is a computer system where we enter the patient's data and the prosthetic prescription. Once the data has been entered, a fibre-optic device will be inserted into the patient's mouth and will take images of it until a complete 3D image is obtained. Together with the data, this 3D image forms a file that is sent to a design programme for personalized adaptation to each patient. The design of the dental elements is carried out using CAD software.

For example, US2018303581 A1 discloses systems and method for fabrication of dental appliances. Said method comprises the steps of receiving data identifying approximate locations of individual teeth in a three-dimensional digital dental model representing an impressioned position of a patient's dentition; generating component models corresponding to individual teeth for each of the identified approximate locations, the component models being disposed at initial positions based on the impressioned position of the patient's dentition; determining target positions for the component models; generating a tooth-positioning appliance design based on the determined target positions for the component models; and causing a tooth-positioning appliance to be fabricated based on the tooth-positioning appliance design.

Currently, the design software known in the dental market is based on the design of mandibular devices through the generation and modification of meshes that define the mouth or dental arches of the patient. A mesh is a data set that defines the surface of the patient's arch in space as a single element. A dental arch is the set of incisor, canine and molar dental pieces that form the arch of the upper and lower jaws of a patient. The aforementioned design software works with a uniform thickness of the complete mesh, which will later be sent to 3D printing and be finished manually.

Once the dental product to be manufactured has been designed, a file is generated to send to the dental printer or 3D printer. In general, the 3D printing process is as follows: the printer, following the instructions from the design file, deposits and solidifies the material. Once the first layer has solidified, it continues with a second layer and repeats the same process, in such a way that the previously designed three-dimensional object is formed layer by layer. However, printed dental objects have been constructed of mesh with a uniform thickness, so that the object must be adjusted manually after printing, modifying and eliminating the excess parts of the material. This adjustment to adapt the device to the patient's mouth is very laborious, and is less detailed in the contact areas between the arches. In other words, the splint that is generated through the aforementioned processes is neither the most accurate nor the most comfortable for the patient.

This invention aims to provide an improved, more accurate method of designing a splint-type intraoral device that provides a more personalized design for greater patient comfort, in addition to minimizing manual adjustments after manufacturing. At the same time, this method of the invention provides the personalised treatment necessary to treat bruxism, sleep apnoea, snoring, tongue function or even create surgical guides.

### Brief description of the invention

The object of the present invention is an improved and more precise method of designing intraoral or mandibular devices, often called dental splints, to treat bruxism, oral respiration, known as snoring, lingual parafunction and sleep apnoea, among other disorders. Specifically, the implementation of the method of the invention will focus on the design of an intraoral device, or splint, without limiting the design method of the invention. The method of the invention is carried out through the use of CAD software installed on a computer, where firstly a file is created based on the intraoral or extraoral scan of the patient's previous models. The file contains the oral data of at least one arch or the previous models of the patient, and is transferred to the CAD software to visualize and work with at least one arch belonging to the patient, in the form of a mesh or single piece. Once the file is opened, the invention design method begins, characterized in that it first comprises a step where the arch mesh is segmented into various dental parts by means of thermal simulation. Thermal simulation consists of assigning heat points of one temperature to dental pieces and heat points of a different temperature to the dental pieces adjacent to the dental pieces with heat points of the first temperature. In this way, adjacent dental pieces have different heat points.

Once the heat points have been assigned to all the dental pieces by means of the CAD software, a thermal simulation is carried out by conduction, so that since heat is not transmitted between adjacent dental pieces as they are at different temperatures, all the dental pieces are defined separately.

Once each dental piece has been defined as individual pieces, the method of the invention continues to define local vectors and centroids to each dental piece, since the design of the device will be outlined one by one, so the local vectors will define the directions of a support curve for generating the final contour of the splint.

The method described has various advantages, such as identifying each and every dental piece identified dental piecein order to satisfy the mechanical and comfort needs of each dental piece for each patient. In addition, it gives more precise control over the design, such as being able to give non-uniform thicknesses throughout the splint. The possibility of designing the splint around each dental piece individually allows the contact surfaces between the opposing arches to be monitored and designed with greater precision and, by means of the design, to control the internal forces of the splint and prevent changes in the patient's bite in the future. In conclusion, the method of the invention makes it possible to design and manufacture personalized intraoral devices for each patient in a more precise way.

Additionally, thanks to the possibility of controlling the amount of material and thickness, it is also possible to adapt intraoral devices to products with less material, to facilitate 3D printing.

### Methods of embodiment of the invention

### Brief description of the figures

The details of the invention can be seen in the accompanying figures, which do not intend to limit the scope of the invention:
- Figure 1 shows an intraoral device (1), splint-type designed and manufactured according to the method of the invention.
- Figure 2 shows a patient's arches (2, 3) in the first step of the method of the invention.
- Figure 3 shows the second step of the method of the invention on an arch (2, 3) to be worked on.
- Figure 3a shows another view of the arch (2, 3) after the second step of the method of the invention.
- Figure 4 shows a view of the software in the third step of the invention.
- Figure 5 shows a view of the software at the start of the fourth step of the invention.
- Figure 6 shows a view of the software for the fourth step of the invention.
- Figure 7 shows a possible embodiment of the method of the invention.
- Figure 8 shows a view of the software for the fifth step of the invention.
- Figure 9 shows another possible embodiment of the method of the invention.
- Figure 10 shows a view after the sixth step of the invention.
- Figure 11 shows another possible embodiment of the method of the invention.
- Figure 12 shows another possible embodiment of the method of the invention.

### Detailed description of the invention

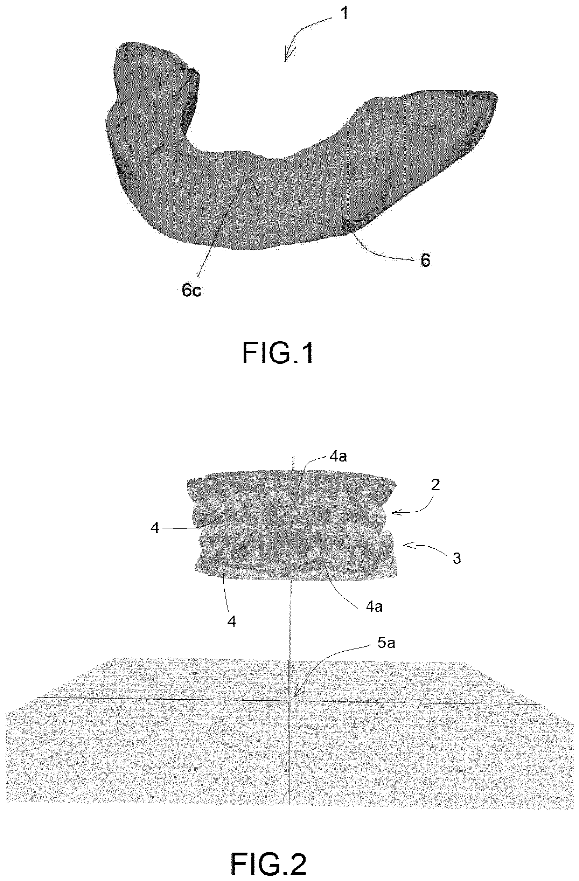

The invention refers to an improved and precise method for the design of intraoral devices (1), such as a dental splint or aligner, like the one seen in Figure 1. Intraoral devices are aimed at dental treatment, where the most common are for the treatment of bruxism and/or sleep apnoea. As in other design methods, the method of the invention can also be applied to the design of other intraoral devices that are based on an intraoral scan of the patient or extraoral scan of the patient's previous models, the subsequent design of the device (1) using a computer and CAD software technology adapted to the patient's dental pieces and finally the 3D printing of the device (1) using CAM technology.

An intraoral scan is based on creating a 3D digital file of the patient's mouth and then working on it. Therefore, for the method of the invention, initially, an intraoral scan of the patient's mouth is carried out to generate at least one file with at least one arch (2, 3) of dental pieces to work with. Normally two files are generated separating the upper arch (2) and the lower arch (3) of the patient. Subsequently, the intraoral scan is transferred to a CAD program or design software using the arch file (2, 3) for which the splint-type intraoral device (1) design is to be configured, adapting it to the dental needs of the patient. As can be seen in Figure 2, when the files are opened in the program, the patient's arch (2, 3) to be worked on is seen as a single piece or mesh with a uniform thickness that is established by the initial data as explained above.

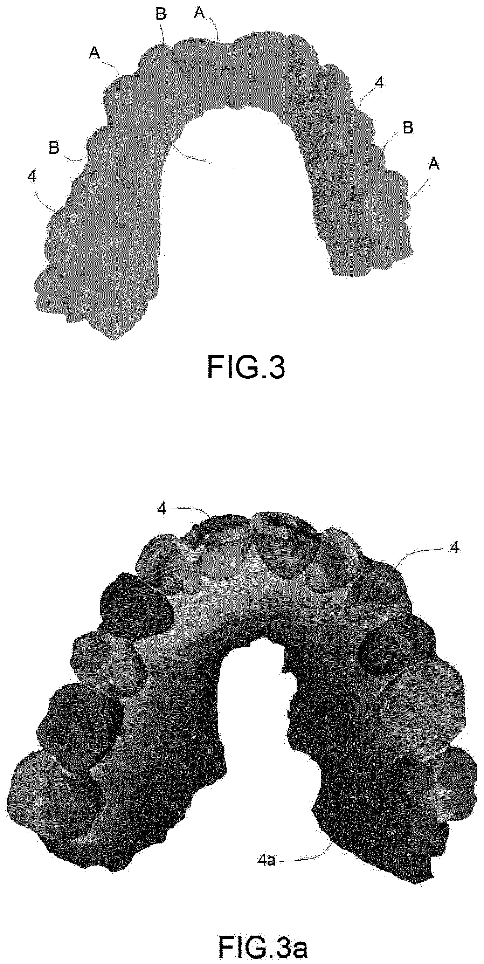

However, the method of the invention is characterized by segmenting the arch (2, 3) defined as a mesh or a single piece, into as many parts as dental pieces (4) that are to be worked on. The segmentation of the arch (2, 3) by means of the CAD software is carried out by means of a thermal simulation, by assigning heat points of temperatures A and B, in the different dental pieces (4) as seen in Figure 3. Heat points of different temperatures are assigned, where certain heat points of one temperature (A) are assigned to some dental pieces (4) and other heat points of another temperature (B) to the dental pieces (4) adjacent to those that comprise the heat points of temperature (A). In this way, the adjacent dental pieces (4) are at different temperatures. In the CAD software, these heat points (A, B) are displayed in different colours, as seen in Figure 3.

These heat points (A, B) are designed to segment the arch (2, 3) on which the work is carried out, defining the different dental pieces (4) of the arch (2, 3) by transmitting heat by conduction. Specifically, the thermal simulation is carried out using the software, and it is can be seen that the heat points (A, B) transmit heat through the mesh to a point that is defined by another temperature, as shown in Figure 3a. In summary, since the adjacent dental pieces (4) comprise heat points (A, B) of different temperatures, the heat is transmitted to where the adjacent dental piece (4) begins, which is defined by another temperature. At that limit where heat is no longer transmitted is the border between the adjacent dental pieces (4), which corresponds to a minimum curvature area. The minimum curvature areas are found in the spaces between the dental pieces (4), and the dental pieces (4) and the gums (4a). In this way, it is possible to isolate the geometry of each dental piece (4) because heat is not transmitted between the different dental pieces (4).

This segmentation makes it possible to isolate the geometry of each dental piece (4) to be able to design the intraoral device (1) around each dental piece (4) individually in a non-uniform way according to the needs of each patient. This method allows the user a very precise manipulation of the intraoral device (1) to be created, satisfying the mechanical and dental needs of each dental piece (4) and providing greater comfort to the patient. In this way, an optimal design can be created to treat any jaw or dental disorder, such as bruxism, sleep apnoea, or to design aligners or surgical guides.

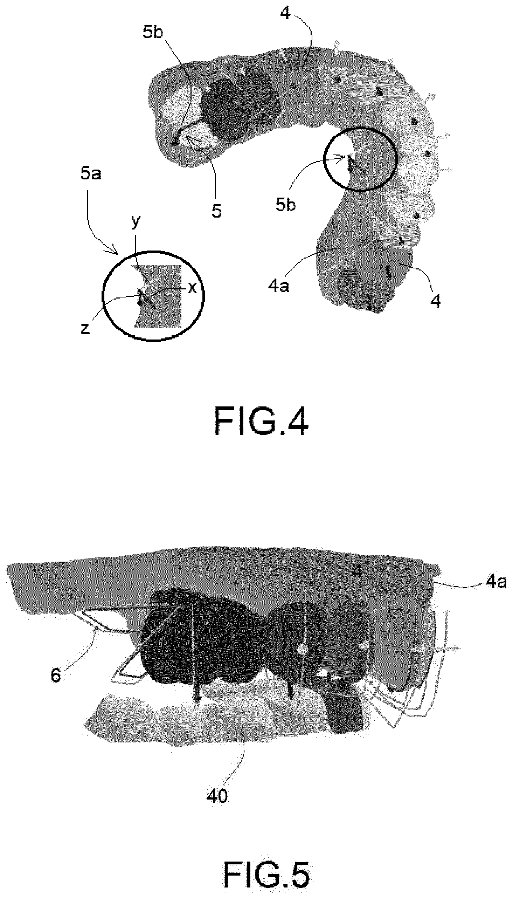

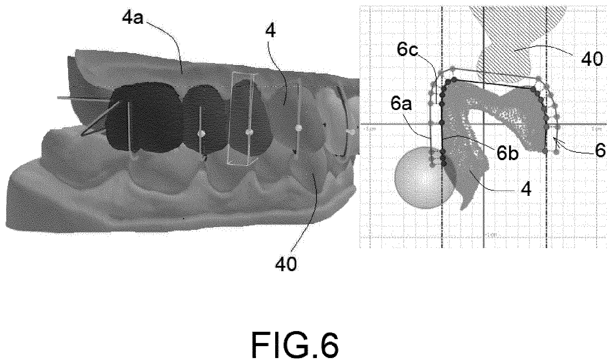

Next, once the dental pieces (4) have been separated, a 3D space is assigned to them, establishing vectors or local axes (5) and centroids (5b) which define the three directions (x, y, z) from the centroid (5b) of each dental piece (4) as seen in Figure 4. A centroid (5b) is understood as the location of the geometric centre of gravity of a body. The local axes (5) of each dental piece (4) will be calculated according to the centroids (5b) of the adjacent dental pieces (4), the bisector between the adjacent centroids (5b) being the direction axis (Y) of each dental piece (4). A bisector is understood as the geometric place of the points of the plane originating at the vertex of an angle and equidistant from the sides of the angle, where the vertex is the centroid (5b) of the analyzed dental piece (4) and the sides of the angle are the lines that join the centroid (5b) of the analyzed dental piece (4) to the adjacent centroids (5b). The direction axis (X) is orthogonal to the direction axis (Y) and the direction axis (Z) is the same as that given in the initial data, i.e., the axis (Z) is parallel to the axis (Z) of the general axes (5a), as seen in Figure 4. These axes (5) will be responsible for defining the directions of a contour (6) of the intraoral device (1) around each dental piece (4) as seen in Figure 5 and 6.

Next, as seen in Figures 5 and 6, in the region corresponding to each dental piece (4) a curve is defined that will serve as a support for the final contour (6) of the splint-type intraoral device (1) according to the method of the invention. The contour surface (6) of the intraoral device (1) is defined by an outer line (6a) defined by the vectors (5) and the surface curve of each dental piece (4) and can be designed and adapted independently. In the next Figure 6, a sectional view of a dental piece (4) in the plane of directions (Y, Z) is shown. The outer line (6a) defines the outer contour (6) of the intraoral device (1) and an inner line (6b) defines the outer surface of the dental piece (4). The distance between the outer line (6a) and an inner line (6b) defines the thickness (6c) of the contour (6) of the intraoral device (1). In this way, each dental piece (4) will have a surface guiding curve, except for the last molars, which can rotate this direction plane (Y, Z) as many degrees as required to obtain the closure of the contour (6) of the intraoral device (1) as seen in Figures 5 and 6. This step allows each section that defines the thickness (6c) of the curve to be adapted and defined to determine the thickness (6c) of the intraoral device (1) dental piece by dental piece, adjusting the distances between the outer line (6a) and the inner line (6b) according to the mechanical or prosthetic needs of the patient. Furthermore, this step makes it possible to monitor the contact surface with the opposing dental piece (40) as shown in Figure 6. This allows the generation of a splint type intraoral device (1), of non-uniform thickness (6c) in the different areas of the splint (1) according to dental diagnosis and treatment with infinite possibilities.

The method of the invention makes it possible to design and produce a splint type intraoral device (1), using independent geometries of the dental pieces (4), instead of using a mesh of the patient's arch (2, 3) as a single piece. In other words, the method generates a surface that is not forced to follow the geometry of the initial data. This highly personalized segmentation and adaptation of the contour (6) of the intraoral device (1) to each dental piece (4) is achieved thanks to the previous step of the method of the invention that has converted a mesh of the patient's arch (2, 3) into several independent dental pieces (4).

Optionally, it is even possible to eliminate the thickness (6c) completely in certain desired contact areas, according to design or dental criteria, so that the contact of the upper arch (2) with the opposing lower arch (3) is dental piece-splint and not splint-splint. This achieves an interrupted intraoral device (1) that provides greater comfort to the patient by reducing the total thickness (6c) of the intraoral device (1) placed in the patient's mouth. Additionally, if the patient is missing a dental piece (4) in one of their arches (2, 3), in the generation of the device (1) the missing piece is filled with material, so the thickness (6c) of the device (1) can be completely eliminated, since it includes additional material, to later carry out other necessary operations.

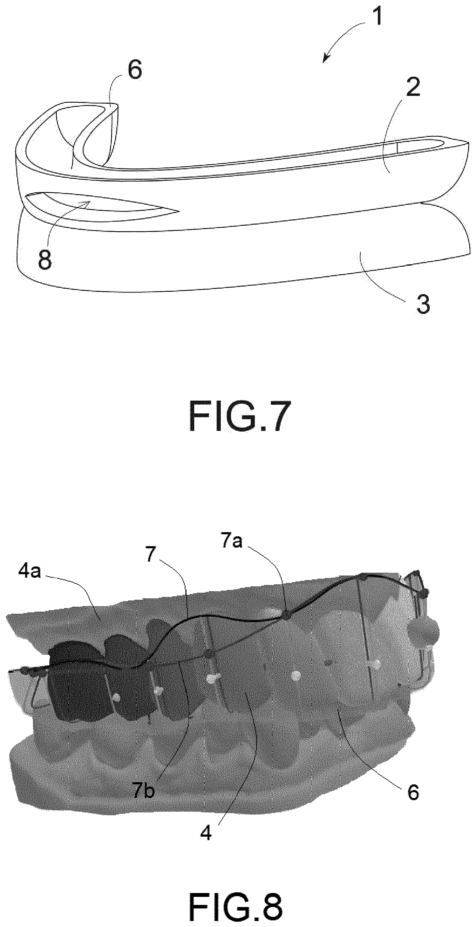

Optionally, Figure 7 shows a possible embodiment of the method of the invention, which makes it possible to eliminate the thickness (6c) of the contour (6) in a frontal area, creating a frontal hole (8) where the incisor dental pieces meet. This front hole (8) makes it possible to reduce the load borne by the incisors, since the front hole (8) makes it possible to transfer this load towards the area of the root of the dental pieces (4). This achieves more comfort for the patient and reduces pain or possible future dental problems. This is an example of how the method of the invention makes it possible to control the action of the internal forces by means of the design of the intraoral device (1), preventing future changes in bite, or inducing them if this is the aim of the treatment.

Once the thicknesses (6c) of the intraoral device (1) have been defined for each dental piece (4) in the arch (2, 3), the next step of the CAD program is to generate a simulation of the final contour (6) of the intraoral device (1), which was defined in the previous step in which there is a limit curve (7) that represents the height of the intraoral device (1) as seen in Figure 8. The next step is to modify the height of the final contour (6) of the intraoral device (1), i.e. defining the height of the intraoral device (1) with respect to each dental piece (4) by transferring some points (7a) on the limit curve (7), called a Spline in CAD design.

The height adjustment is carried out by transferring some points (7a) of the limit curve (7) or spline from each dental piece (4) to a desired height, defined by a final curve (7b) as shown in Figure 8. It is possible to adjust the height of the points (7a) both in the vestibular area and in the lingual area (inside the mouth) to adjust the height of the intraoral device (1) with respect to the criteria or needs of each patient. The height of the splint is recommended to be above the equator of the dental piece to ensure minimal retention when placed in the patient's mouth. In this way, the height of the intraoral device (1) in each section of dental pieces or dental pieces (4) can be defined according to the criteria or dental or treatment needs of the patient.

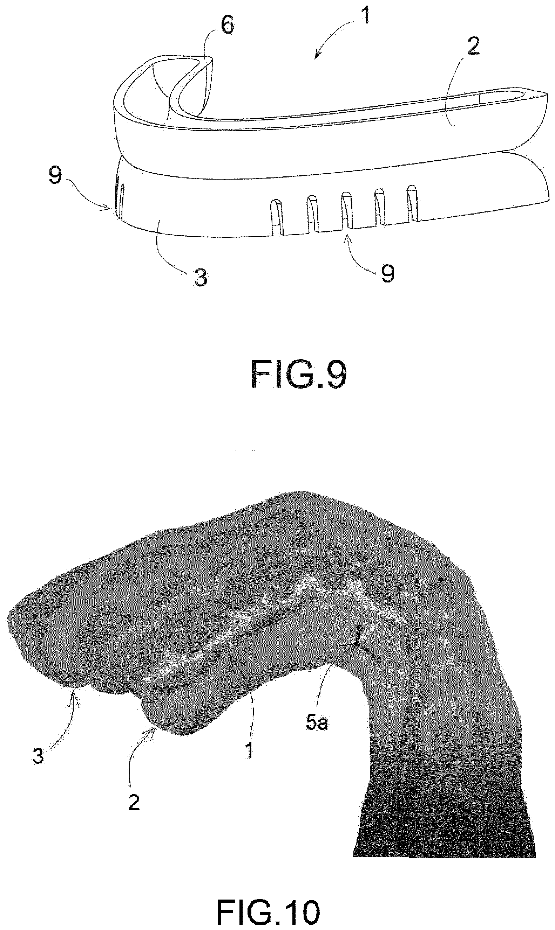

In addition, alternatively, another possible embodiment of the method of the invention consists of modifying the height of the intraoral device (1) in at least one interproximal area, defined as the space formed between the dental pieces, which is occupied by the gum (4a). This modification consists of moving the points (7a) of the limit curve (7) to a line below the equator of the dental piece, generating cuts or vertical grooves (9) to ensure the retention of the intraoral device (1) in the arch (2, 3) with a more comfortable insertion force, as shown in Figure 9.

Once the thickness (6c) and height of the intraoral device (1) have been adjusted, the final contour (6) is generated in each dental piece (4) and the design made in the CAD program is accepted. Subsequently, all the fractions of the intraoral device (1), i.e. all the geometries of each dental piece (4), are joined together to convert the intraoral device (1) into a mesh again and work from now on with the intraoral device (1) as a whole. Once the mesh of the intraoral device (1) has been generated, the intersection of the arches (2, 3) with the opposing arch (2, 3) is calculated, to be able to define the amount of material desired in the contact areas between the arches (2, 3) according to the needs of the patient.

As can be seen in Figure 10, the next step of the method, after converting the intraoral device (1) into a mesh, is to map the intraoral device (1) to visualize the contact areas between the arches (2, 3). Normally, in design software, the contacts between arches (2, 3) are defined with a gradient of colours defined by at least three shades according to the contact or intersection of the arches (2, 3) with the opposing arch. Generally, the colour gradient is red-orange-green according to the degree of intersection as shown in Figure 10. In this way, it is possible to visualize where the amount of material in the contact areas that will bother the patient can be reduced or increased, simply by indicating the areas and choosing whether to add or remove material according to criteria established in the software tools. Through this step, it is possible to adapt the contact areas of the intraoral device (1) between the dental arches (2, 3), achieving the right occlusal adjustment without having to print and mould it manually.

Once the amount of material in the intraoral device (1) has been adapted to the prosthetic needs of the patient and the previous step has been validated, the penultimate step consists of performing Boolean operations. Boolean operations are defined as operations to create an object by combining two of them through a mathematical operation, where the two objects can be subtracted, joined or intersected to form the new object. In the design sector using CAD, this is a technique used in 3D, using planes, surfaces or solids to obtain volumes from the addition, subtraction or intersection of other volumes.

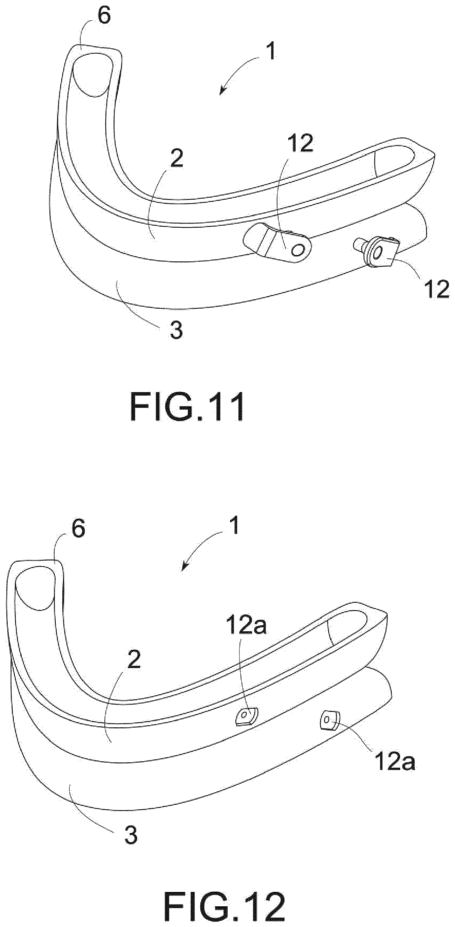

Optionally, a possible embodiment of the method of the invention by means of Boolean operations makes it possible to generate housings in the contact areas between arches (2, 3), similar to holes (12a) such as those seen in Figure 12. Inside the aforementioned housings there are buttons made of resistant material such as titanium or wire. These buttons inserted into the intraoral device (1) allow the reinforcement of the areas where the housings are made, and provide greater hardness and resistance in the contact points or areas of strong bites between the arches (2, 3). In this way, instead of adding material or thickness (6c) to the contour (6), the intraoral device (1) obtains greater robustness with a lower thickness (6c) of the contour (6). In this way, the stiffness of the intraoral device (1) can be controlled and the result is a comfortable bite and a strong intraoral device (1). Alternatively, buttons made of elastic material can also be inserted into the housings for treatments that require greater flexibility in the bite. In short, these alternative operations make it possible to gain resistance and flexibility in the design.

Additionally, for sleep apnoea treatments, it is necessary to connect mandibular advancement mechanisms, which are fixed to the sides of the upper (2) and lower (3) arches of the patient, positioned and tensed in such a way that they keep the lower arch (3) advanced with respect to the upper arch (2). Another possible embodiment of the method of the invention makes it possible to fix the mandibular advancement mechanisms or any other orthodontic system in the intraoral device (1) by generating projections (12) such as those shown in Figure 11 or gaps (12a) intended to place adhesive as shown in Figure 12, to hold the projections (12) as independent parts.

Another possible Boolean operation of the design method of the invention consists of generating guide holes in the splint-type device (1) in those areas of the splint where the patient is missing a dental piece (4). These holes intended to place a device that is metallic or of another material which serves for inserting and guiding the drilling tools used with a surgical guide, for example for the placement of an implant or other similar application.

Another possible Boolean operation of the method of the invention is based on generating a reticulated structure on the inner surface of the intraoral device (1). The internal reticulated structure is created from a geometric pattern on an YZ plane that is subsequently adjusted to the internal geometry of the intraoral device (1) through a transformation of curved coordinates. In this way, an intraoral device (1) is obtained with an irregular interior, with less rigidity, using less material and at the same time giving greater comfort to the patient.

Another possible Boolean operation of the method of the invention consists of selectively adding predefined protruding shapes to the splint (1), such as conical shapes, in the interior part of the upper or lower arch (2, 3) with certain functions, for example, educating the tongue so that it does not position itself or press in areas where it should not. In other words, the patient who presses or positions the tongue against the incisors, notices an uncomfortable surface, in such a way as to avoid that position. This makes it possible to prevent parafunctional habits, which are all jaw movements that have no functional purpose.

All these possibilities of the design method of the invention allow for the precise generation of an intraoral (1) or mandibular device for the treatment of each patient and with savings in material, since all corrections are made by CAD, facilitating 3D printing.

Finally, to complete the method and use of the CAD design program, the dental parts (4) are emptied to observe the intraoral device (1) as seen in Figure 1 and the entry-exit path is defined for the intraoral device (1) placed in the patient's mouth, where the pathway may be irregular. In the case of the invention, it consists of generating the negative of the patient's arch (2, 3) in the intraoral device (1) to simulate the entry of the patient's real arch (2, 3) into the intraoral device (1), as would be done in the surgery by the dentist and the patient, so that the splint (1) can fit into the patient's mouth easily and comfortably.

It is also recommendable to perform a simulation of jaw movement to observe if the intraoral device (1) intersects or achieves the desired treatment. This simulation allows the design to be rectified if necessary before sending it to the 3D printer.

Once the design of the intraoral device (1) is finished using the computer CAD program, a file is generated with all the necessary data to be sent to the 3D printer or other CAM device, for the manufacture of the device (1).

## Claims

1. Design method of an intraoral device (1), splint type, through the use of CAD software, where a file based on the intraoral scan or scan of the previous models of a patient is opened and the data of at least one arch (2, 3) of the patient in the form of a mesh or piece, where the design method is **characterized by** comprising the steps of:
- segmenting the arch mesh (2, 3) into the different dental pieces (4) by means of a thermal simulation by assigning heat points of a temperature (A) to dental pieces (4) and points of heat of another temperature (B) to the dental pieces (4) adjacent to those that comprise heat points of temperature (A), so that the adjacent dental pieces (4) have different heat points (A, B),
- defining at least some local vectors (5) and centroids (5b) to each dental piece (4), which will define the directions of a support curve of a final contour (6) of the intraoral device (1),

2. Design method of an intraoral device (1), according to claim 1, **characterized in that** it comprises the additional steps of;
- modifying at least one thickness (6c) of the contour (6) of at least one dental piece (4) individually,
- generating a simulation of the final contour (6) of the intraoral device (1), where a limit curve (7) is generated to represent the height of the intraoral device (1) in each dental piece (4),
- modifying the height of the device (1) by moving some points (7a) of the limit curve (7) defined in each dental piece (4) towards a final curve (7b) according to the prosthetic treatment needs of the patient,

3. Design method of an intraoral device (1), according to claim 1, **characterized in that** the segmentation of the mesh is carried out by transmitting heat by conduction to areas of minimum radii of curvature between the different dental pieces (4).

4. Design method of an intraoral device (1), according to claim 2, **characterized in that** the modification of the thickness (6c) comprises the additional step of adjusting the distance between an outer line (6a) and an inner line ( 6b) that define the thickness (6c) of each dental piece (4).

5. Design method of an intraoral device (1), according to claim 2, **characterized in that** the modification of the thickness (6c) comprises the additional step of completely eliminating the thickness (6c) in at least one contact area, so that the contact of the intraoral device (1) of the upper arch (2) with that of the opposing lower arch (3) is dental piece-splint and not splint-splint.

6. Design method of an intraoral device (1), according to claim 2, **characterized in that** the modification of the thickness (6c) allows a front hole (8) to be made in the front area of the intraoral device (1) where the incisor dental pieces are located.

7. Design method of an intraoral device (1), according to claim 2, **characterized in that** the modification of the limit curve (7) that defines the height of the intraoral device (1) comprises the additional step of transferring the points (7a) from the limit curve (7) to a final curve (7b) below the equator of the dental piece (4) generating vertical cuts or grooves (9).

8. Design method of an intraoral device (1), according to claim 1, **characterized in that** it comprises the additional steps of;
- joining the individual geometries of each dental piece (4) of the splint-type intraoral device (1) to convert the final contour (6) into a mesh or single piece,
- removing or adding material from the final contour (6) mesh of the splint-type intraoral device (1) and,
- generating the file with a splint-type device (1) designed to send to the 3D printer or another CAM device, for manufacturing.

9. Design method of an intraoral device (1), according to claim 8, **characterized in that** additional Boolean operations are performed during the step of adding or removing mesh material.

10. Design method of an intraoral device (1), according to claim 9, where the Boolean operation consists of creating housings where buttons of resistant material such as titanium or wire are fixed to provide greater hardness and resistance in the contact areas or areas of strong bites between the patient's dental arches (2, 3) with a lesser thickness (6c) of the device (1).

11. Design method of an intraoral device (1), according to claim 9, where the Boolean operation consists of creating housings where buttons of elastic material are attached for treatments that require greater flexibility in the bite of the patient's mouth and a lesser thickness (6c) of the intraoral device (1).

12. Design method of an intraoral device (1), according to claim 9, where the Boolean operation consists of generating projections (12) in the splint (1) to fix mandibular advancement mechanisms or other orthodontic systems.

13. Design method of an intraoral device (1), according to claim 9, where the Boolean operation consists of generating holes (12a) in the splint (1) intended to place adhesive to hold the aforementioned projections (12) as independent pieces, where the mandibular advancement mechanisms are fixed.

14. Design method of an intraoral device (1), according to claim 9, where the Boolean operation consists of generating guide holes in the splint-type device (1) intended to place a metal device or other material that serves to guide the milling tools used with a surgical guide or other similar application.

15. Design method of an intraoral device (1), according to claim 9, where the Boolean operation consists of generating a reticulated structure on the inner surface of the splint-type device (1) that is created from a geometric pattern on a YZ plane, which by means of a curved coordinate transformation is adjusted to the internal geometry of the splint-type device (1).

## Patentansprüche

1. Entwurfsverfahren einer intraoralen Vorrichtung (1) vom Typ Schiene durch die Verwendung von CAD-Software, wobei eine Datei auf der Grundlage des intraoralen Scans oder des Scans der vorherigen Modelle eines Patienten geöffnet wird und die Daten mindestens eines Bogens (2, 3) des Patienten in Form einer Vermaschung oder Teils, wobei das Entwurfsverfahren **dadurch gekennzeichnet ist, dass** es die folgenden Schritte umfasst:
- Segmentierung der Bogenvermaschung (2, 3) in die verschiedenen Zahnteile (4) durch eine thermische Simulation, indem den Zahnteilen (4) Wärmepunkte einer Temperatur (A) und den Zahnteilen (4), die an die Wärmepunkte der Temperatur (A) angrenzen, Wärmepunkte einer anderen Temperatur (B) zugeordnet werden, so dass die angrenzenden Zahnteile (4) unterschiedliche Wärmepunkte (A, B) aufweisen,
- Definition mindestens einiger lokaler Vektoren (5) und Zentroiden (5b) zu jedem Zahnteil (4), die die Richtungen einer Stützkurve einer Endkontur (6) der intraoralen Vorrichtung (1) festlegen,

2. Entwurfsverfahren einer intraoralen Vorrichtung (1) nach Anspruch 1, **dadurch gekennzeichnet, dass** es die folgenden zusätzlichen Schritte umfasst;
- Einzelmodifizierung mindestens einer Dicke (6c) der Kontur (6) von mindestens einem Zahnteil (4),
- Erzeugen einer Simulation der Endkontur (6) der intraoralen Vorrichtung (1), wobei eine Grenzkurve (7) erzeugt wird, um die Höhe der intraoralen Vorrichtung (1) in jedem Zahnteil (4) darzustellen,
- Modifizierung der Höhe der Vorrichtung (1) durch Verschieben einiger Punkte (7a) der Grenzkurve (7), die in jedem Zahnteil (4) definiert ist, in Richtung einer endgültigen Kurve (7b) entsprechend den prothetischen Behandlungserfordernissen des Patienten,

3. Entwurfsverfahren einer intraoralen Vorrichtung (1) nach Anspruch 1, **dadurch gekennzeichnet, dass** die Segmentierung der Vermaschung durch Wärmeübertragung durch Konduktion auf Bereiche mit minimalen Krümmungsradien zwischen den verschiedenen Zahnteilen (4) erfolgt.

4. Entwurfsverfahren einer intraoralen Vorrichtung (1) nach Anspruch 2, **dadurch gekennzeichnet, dass** die Modifizierung der Dicke (6c) den zusätzlichen Schritt der Einstellung des Abstandes zwischen einer äußeren Linie (6a) und einer inneren Linie (6b) umfasst, die die Dicke (6c) jedes Zahnteils (4) festlegen.

5. Entwurfsverfahren einer intraoralen Vorrichtung (1) nach Anspruch 2, **dadurch gekennzeichnet, dass** die Modifizierung der Dicke (6c) den zusätzlichen Schritt der vollständigen Eliminierung der Dicke (6c) in mindestens einem Kontaktbereich umfasst, so dass der Kontakt der intraoralen Vorrichtung (1) des oberen Bogens (2) mit dem des gegenüberliegenden unteren Bogens (3) Zahnteil-Schiene und nicht Schiene-Schiene ist.

6. Entwurfsverfahren einer intraoralen Vorrichtung (1) nach Anspruch 2, **dadurch gekennzeichnet, dass** die Modifizierung der Dicke (6c) es ermöglicht, im vorderen Bereich der intraoralen Vorrichtung (1), wo sich die Zahnteile des Schneidezahns befinden, eine vordere Aussparung (8) herzustellen.

7. Entwurfsverfahren einer intraoralen Vorrichtung (1) nach Anspruch 2, **dadurch gekennzeichnet, dass** die Modifikation der Grenzkurve (7), die die Höhe der intraoralen Vorrichtung (1) festlegt, den zusätzlichen Schritt umfasst, die Punkte (7a) von der Grenzkurve (7) in eine Endkurve (7b) unterhalb des Äquators des Zahnteils (4) zu übertragen, wobei vertikale Schnitte oder Rillen (9) erzeugt werden.

8. Entwurfsverfahren einer intraoralen Vorrichtung (1) nach Anspruch 1, **dadurch gekennzeichnet, dass** es die folgenden zusätzlichen Schritte umfasst;
- Zusammenfügen der einzelnen Geometrien der einzelnen Zahnteile (4) der intraoralen Vorrichtung (1) vom Typ Schiene, um die Endkontur (6) in eine Vermaschung oder ein einziges Teil umzuwandeln,
- Entfernen oder Hinzufügen von Material aus der Vermaschung der Endkontur (6) der intraoralen Vorrichtung (1) vom Typ Schiene und,
- Erstellung der Datei mit einer Vorrichtung (1) vom Typ Schiene, das für die Weiterleitung an einen 3D-Drucker oder ein anderes CAM-Gerät bestimmt ist, für die Fertigung.

9. Entwurfsverfahren einer intraoralen Vorrichtung (1) nach Anspruch 8, **dadurch gekennzeichnet, dass** zusätzliche Boolesche Operationen während des Schrittes des Hinzufügens oder Entfernens von Vermaschungsmaterial durchgeführt werden.

10. Entwurfsverfahren einer intraoralen Vorrichtung (1) nach Anspruch 9, wobei die Boolesche Operation darin besteht, Gehäuse zu schaffen, an denen Knöpfe aus widerstandsfähigem Material wie Titan oder Draht befestigt werden, um eine größere Härte und Widerstandsfähigkeit in den Kontaktbereichen oder Bereichen starker Bisse zwischen den Zahnbögen (2, 3) des Patienten mit einer geringeren Dicke (6c) der Vorrichtung (1) zu gewährleisten.

11. Entwurfsverfahren einer intraoralen Vorrichtung (1) nach Anspruch 9, wobei die Boolesche Operation darin besteht, Gehäuse zu schaffen, an denen Knöpfe aus elastischem Material befestigt werden, für Behandlungen, die eine größere Flexibilität im Biss des Patienten und eine geringere Dicke (6c) der intraoralen Vorrichtung (1) erfordern.

12. Entwurfsverfahren einer intraoralen Vorrichtung (1) nach Anspruch 9, wobei die Boolesche Operation darin besteht, Vorsprünge (12) in der Schiene (1) zu erzeugen, um Unterkiefervorschubmechanismen oder andere kieferorthopädische Systeme zu befestigen.

13. Entwurfsverfahren einer intraoralen Vorrichtung (1) nach Anspruch 9, wobei die Boolesche Operation darin besteht, in der Schiene (1) Hohlräume (12a) zu erzeugen, die dazu bestimmt sind, Klebstoff zu platzieren, um die vorgenannten Vorsprünge (12) als unabhängige Teile zu halten, an denen die Unterkiefervorschubmechanismen befestigt sind.

14. Entwurfsverfahren einer intraoralen Vorrichtung (1) nach Anspruch 9, wobei die Boolesche Operation darin besteht, in der Vorrichtung (1) vom Typ Schiene Führungshohlräume zu erzeugen, die dazu bestimmt sind, eine Metallvorrichtung oder ein anderes Material zu platzieren, das dazu dient, die mit einer chirurgischen Führung oder einer anderen ähnlichen Anwendung verwendeten Fräswerkzeuge zu führen.

15. Entwurfsverfahren einer intraoralen Vorrichtung (1) nach Anspruch 9, wobei die Boolesche Operation darin besteht, auf der Innenfläche der Vorrichtung (1) vom Typ Schiene eine netzartige Struktur zu erzeugen, die aus einem geometrischen Muster auf einer YZ-Ebene erzeugt wird, das mittels einer gekrümmten Koordinatentransformation an die innere Geometrie der Vorrichtung (1) vom Typ Schiene angepasst wird.

## Revendications

1. Méthode de design d'un dispositif intrabuccal (1), de type goupille, par un logiciel de CAO, où un fichier basé sur le scan intrabuccal ou le scan des modèles précédents d'un patient est ouvert et les données d'au moins une arcade (2, 3) du patient sous forme de maille ou de pièce, où la méthode de design se **caractérise par le fait que** les étapes qu'elle comprend sont :
- segmentation de la maille de l'arcade (2, 3) dans les différentes pièces dentaires (4) par une simulation thermique en attribuant des points de chaleur d'une température (A) aux pièces dentaires (4) et des points de chaleur d'une autre température (B) aux pièces dentaires (4) adjacentes à celles qui comprennent des points de chaleur de température (A), de telle sorte que les pièces dentaires adjacentes (4) ont différents points de chaleur (A, B)
- en définissant au moins quelques vecteurs locaux (5) et centroïdes (5) pour chaque pièce dentaire (4), qui composeront les directions d'une courbe de support d'un contour final (6) du dispositif intrabuccal (1),

2. Méthode de design d'un dispositif intrabuccal (1), conformément à la revendication 1, **caractérisée par le fait qu'**elle comprend d'autres étapes supplémentaires :
- modification d'au moins une épaisseur (6c) du contour (6) d'au moins une pièce dentaire (4) individuellement,
- création d'une simulation du contour final (6) du dispositif intrabuccal (1), où une courbe limite (7) est créée pour représenter la hauteur du dispositif intrabuccal (1) sur chaque pièce dentaire (4),
- modification de la hauteur du dispositif (1) en déplaçant certains points (7a) de la courbe de limite (7) définie dans chaque pièce dentaire (4) vers une courbe finale (7b) en fonction des besoins de traitement prothétique du patient.

3. Méthode de design d'un dispositif intrabuccal (1), conformément à la revendication 1, **caractérisée par le fait que** la segmentation de la maille est réalisée en transmettant la chaleur par conduction à des zones de rayons de courbure minimaux entre les différentes pièces dentaires (4).

4. Méthode de design d'un dispositif intrabuccal (1), conformément à la revendication 2, **caractérisée par le fait que** la modification de l'épaisseur (6c) comprend l'étape supplémentaire de réglage de la distance entre une ligne extérieure (6a) et une ligne intérieure (6b) définissant l'épaisseur (6c) de chaque pièce dentaire (4).

5. Méthode de design d'un dispositif intrabuccal (1), conformément à la revendication 2, **caractérisée par le fait que** la modification de l'épaisseur (6c) comprend l'étape supplémentaire d'élimination totale de l'épaisseur (6c) sur au moins une zone de contact, de telle sorte que le contact du dispositif intrabuccal (1) de l'arcade supérieure (2) avec celui de l'arcade inférieure opposée (3) est une pièce dentaire-goupille et non une goupille-goupille.

6. Méthode de design d'un dispositif intrabuccal (1), conformément à la revendication 2, **caractérisée par le fait que** la modification de l'épaisseur (6c) permet de percer un trou frontal (8) dans la zone avant du dispositif intrabuccal (1) où sont placées les pièces dentaires incisives.

7. Méthode de design d'un dispositif intrabuccal (1), conformément à la revendication 2, **caractérisée par le fait que** la modification de la courbe limite (7) définissant la hauteur du dispositif intrabuccal (1) comprend l'étape supplémentaire de transfert des points (7a) de la courbe limite (7) à une courbe finale (7) sous l'équateur de la pièce dentaire (4) créant des entailles ou des rainures verticales (9).

8. Méthode de design d'un dispositif intrabuccal (1), conformément à la revendication 1, **caractérisée par le fait qu'**elle comprend d'autres étapes supplémentaires qui sont les suivantes :
- union des géométries individuelles de chaque pièce dentaire (4) du dispositif intrabuccal de type goupille (1) pour transformer le contour final (6) en une maille ou une pièce unique,
- retrait ou ajout de matière sur la maille du contour final (6) du dispositif intrabuccal de type goupille (1) et
- création du fichier avec un dispositif de type goupille (1) conçu pour être envoyé à l'imprimante 3D ou à un autre dispositif CAM, pour la fabrication.

9. Méthode de design d'un dispositif intrabuccal (1), conformément à la revendication 8, **caractérisée par le fait que** des opérations booléennes supplémentaires sont réalisées pendant l'ajout ou le retrait du matériau de la maille.

10. Méthode de design d'un dispositif intrabuccal (1), conformément à la revendication 9, dans laquelle l'opération booléenne consiste en la création de logements dans lesquels des boutons réalisés en un matériau résistant comme le titane ou du fil sont fixés pour renforcer la dureté et apporter une meilleure résistance dans les zones de contact ou des morsures fortes entre les arcades dentaires du patient (2, 3) avec une moindre épaisseur (6c) du dispositif (1).

11. Méthode de design d'un dispositif intrabuccal (1), conformément à la revendication 9, dans laquelle l'opération booléenne consiste en la création de logements dans lesquels sont fixés des boutons en matériau élastique pour les traitements nécessitant une meilleure flexibilité dans l'occlusion de la bouche du patient et une épaisseur réduite (6c) du dispositif intrabuccal (1).

12. Méthode de design d'un dispositif intrabuccal (1), conformément à la revendication 9, dans laquelle l'opération booléenne consiste en la création de saillies (12) dans la goupille (1) pour fixer des mécanismes d'avancement mandibulaire ou d'autres systèmes orthodontiques.

13. Méthode de design d'un dispositif intrabuccal (1), conformément à la revendication 9, dans laquelle l'opération booléenne consiste en la perforation d'orifices (12a) dans la goupille (1) prévus pour loger l'adhésif maintenant les projections (12) susmentionnées comme des pièces indépendantes, où sont fixés les mécanismes d'avancement mandibulaire.

14. Méthode de design d'un dispositif intrabuccal (1), conformément à la revendication 9, dans laquelle l'opération booléenne consiste en la perforation d'orifices de guidage dans le dispositif de type goupille (1) destinés à accueillir un dispositif métallique ou d'un autre matériau servant à guider les outils de fraisage utilisés avec un guide chirurgical ou une autre application similaire

15. Méthode de design d'un dispositif intrabuccal (1), conformément à la revendication 9, dans laquelle l'opération booléenne consiste en la création d'une structure réticulée sur la surface interne du dispositif de type goupille (1) qui est créée à partir d'un patron géométrique sur un plan YZ, qui, via une transformation de coordonnées courbes, est ajustée à la géométrie interne du dispositif de type goupille (1).
